# EUROPEAN PATENT SPECIFICATION

(11) **EP 3 294 644 B1**
(45) Date of publication and mention of the grant of the patent: **11.03.2020**
(21) Application number: 16796927.8
(22) Date of filing: 04.05.2016
(51) Int. Cl.: G07F 11/24

(54) **EARPLUG DISPENSER HAVING A HAPTIC FEEDBACK**
OHRSTÖPSELAUSGABEVORRICHTUNG MIT HAPTISCHER RÜCKMELDUNG
DISTRIBUTEUR DE BOUCHON D'OREILLE AYANT UNE RÉTROACTION HAPTIQUE

(30) Priority: 15.05.2015 US 201562161976 P
(43) Date of publication of application: 21.03.2018
(73) Proprietor: 3M Innovative Properties Company, St. Paul, MN 55133-3427 (US)
(72) Inventor: ZILLIGEN, Guenter M., 41453 Neuss (DE); RECHLITZ, Karl-Heinz, 41453 Neuss (DE); RUDEK, David M., 41453 Neuss (DE)
(74) Representative: Mathys & Squire
(86) International application number: PCT/US2016/030743
(87) International publication number: WO 2016/186845

(56) References cited:
- WO-A1-2011/026239
- WO-A1-2013/030547
- US-A- 5 259 532
- US-A1- 2008 116 219
- US-B1- 6 299 019

## Description

### Technical Field

The present disclosure relates to product dispensers. More particularly, the present disclosure relates to manually operable dispensers providing a haptic feedback to a user.

### Background

Disposable earplugs are routinely used in a variety of settings. In many instances, such as industrial or manufacturing environments, it is desirable to have a large quantity of disposable earplugs readily available at all times. Conventionally, disposable earplugs have been made available in bulk form, for example, by way of a large open box in which the earplugs are loosely maintained. Users simply reach into the box and retrieve earplugs as needed.

Various disposable earplug dispensers have also been devised. Typically, a dispenser loosely stores a large, bulk supply of disposable earplugs and provides a manually operable mechanism intended to dispense or vend individual earplugs. The dispensing mechanism may include a wheel having a series of discrete holes such that earplugs fall into respective holes and are individually dispensed from the mechanism, under the force of gravity, with rotation of the wheel. Notably, rotary dispensing mechanisms sometimes employed with earplug dispensers are akin to those found with some medication capsule dispensers or other devices intended to store and individually dispense (or vend) small, hard objects (e.g., gumballs). However, certain physical characteristics unique to disposable earplugs present distinct concerns not fully addressed by conventional rotary-type dispensing mechanisms.

For example, some types of disposable earplugs are formed of a slow-recovery foam material, open cell or closed surface and, unlike hard objects, are compressible. Further, most disposable earplugs exhibit some degree of tackiness at their outer surface. These unique characteristics make it difficult for a conventional rotary-type dispensing mechanism to accurately and consistently dispense only a single earplug with each user-caused wheel rotation. Further, malfunctions can be prevalent, with the compressible earplugs easily becoming lodged between various moving components of the dispensing mechanism.

Document US6299019 B1 discloses an earplug dispenser which can receive a container with many earplugs and dispense them one or two at a time as a person turns a handle. Document WO2013/030547 A1 discloses a dispenser for dispensing of items, especially solid items, particularly non-extrudable solid items, featuring recesses or voids, including annular items, comprising means for providing rotation feedback to a user of the dispenser, which may comprise a momentary increase friction/resistance during rotation.

### Summary

The present disclosure provides, in an exemplary embodiment, a manually operable dispenser for dispensing earplugs including a dispensing mechanism defining a longitudinal axis. The dispensing mechanism includes a separator assembly defining a well and one or more bores each open to the well, an index assembly including a handle and a plate connected to the handle defining a dispensing aperture, the index assembly rotatably coupled with the separator assembly such that individual bores may be selectively aligned with the dispensing aperture by user rotation of the handle, a first dispensing mechanism portion, and a second dispensing mechanism portion that interacts with the first dispensing mechanism portion during rotation of the handle. Interaction between the first dispensing mechanism portion and the second dispensing mechanism affects a rotational resistance (τ) at the handle such that rotational resistance (τ) varies between a maximum rotational resistance (τmax) and a minimum rotational resistance (τmin) as the handle is rotated by a user.

In another exemplary embodiment, a manually operable dispenser for dispensing earplugs is provided, including a dispensing mechanism defining a longitudinal axis. The dispensing mechanism includes a separator assembly defining a well and a plurality of bores each open to the well and having an annular hub including one or more stiffening elements, and an index assembly including a handle, a plate connected to the handle defining a dispensing aperture, and one or more lobes protruding from a peripheral edge of the plate. The index assembly is rotatably coupled to the separator assembly such that the dispensing aperture may be selectively aligned with individual bores by a manually-applied rotational force. The plate is positioned within the annular hub and the one or more lobes are in at least intermittent contact with the annular hub such that dispensing mechanism exhibits a first rotational resistance (τ1) at the handle when the dispensing aperture is aligned with one of the plurality of bores, and a second rotational resistance at the handle when the dispensing aperture is not aligned with one of the plurality of bores, and (τ2) > (2^{∗}τ1).

The above summary is not intended to describe each disclosed embodiment or every implementation. The Figures and Detailed Description, which follow, more particularly exemplify illustrative embodiments.

### Brief Description of Drawings

The present description will be further explained with reference to the appended Figures, wherein like structure is referred to by like numerals throughout the several views, and wherein:
FIG. 1A is a perspective, exploded view of an exemplary earplug dispenser according to the present description;
FIG. 1B is a perspective view of the exemplary dispenser of FIG. 1A upon final assembly, along with the container;
FIG. 2 is a perspective, exploded view of an exemplary dispensing mechanism according to the present disclosure and useful with the dispenser of FIG. 1A, including a separator assembly and an index assembly;
FIG. 3A is a top perspective view of the exemplary separator assembly of FIG. 2;
FIG. 3B is a rear perspective view of the exemplary separator assembly of FIG. 3A;
FIG. 4A is a simplified side view of an exemplary earplug;
FIGS. 4B and 4C are enlarged, cross-sectional views of a portion of the exemplary separator assembly of FIG. 3A, and illustrate an interface between a disposable earplug and a bore provided with the separator assembly;
FIG. 5 is a cross-sectional view of an exemplary handle portion useful with the index assembly of FIG. 2;
FIG. 6 is a top perspective view of an exemplary mixing body useful with the index assembly of FIG. 2;
FIG. 7 is a cross-sectional view of the exemplary dispensing mechanism of FIG. 2.
FIG. 8 is a representative force profile of an exemplary dispensing mechanism according to the present description.
FIGS. 9A-9D illustrate operation of the exemplary dispensing mechanism of FIG. 2 in handling disposable earplugs prior to dispensing the earplugs
FIG. 10 is a perspective, exploded view of another exemplary dispensing mechanism according to the present disclosure;
FIG. 11A is a perspective, exploded view of a portion of the exemplary dispenser of FIG. 1A, including the exemplary dispensing mechanism of FIG. 2 and a container;
FIG. 11B is a cross-sectional view of a portion of the exemplary dispensing mechanism and container of FIG. 11A upon final assembly;
FIG. 11C is a cross-sectional view of the exemplary assembly of FIG. 11B and loaded with disposable earplugs;
FIG. 12A is an exploded, perspective view of an exemplary dispensing unit useful with the dispenser of FIG. 1A, including the exemplary dispensing mechanism of FIG. 2 and a frame;
FIG. 12B is a top plan view of the components of FIG. 12A upon final assembly; and
FIG. 13 is a side view of portions of the exemplary dispenser of FIG. 1A, including a dispensing mechanism, a frame, and a stand.

While the above-identified figures set forth various embodiments of the disclosed subject matter, other embodiments are also contemplated. In all cases, this description presents the disclosed subject matter by way of representation and not limitation. It should be understood that numerous other modifications and embodiments can be devised by those skilled in the art which fall within the scope of the principles of this description.

### Detailed Description

The present disclosure provides a manually operable dispenser that may be used to dispense earplugs. An exemplary dispenser includes a dispensing mechanism defining a longitudinal axis and having a separator assembly defining a well and a plurality of bores each open to the well, and an index assembly. The index assembly includes a handle and a plate connected to the handle defining a dispensing aperture, such that the dispensing aperture may be selectively aligned with individual bores when a user rotates the handle to allow individual earplugs to be dispensed. The dispensing mechanism further includes a first dispensing mechanism portion and a second dispensing mechanism portion that interact with each other during rotation of the handle. Interaction between the first dispensing mechanism portion and second dispensing mechanism portion is configured to affect a rotational resistance at the handle. Rotational resistance may cause a user to rotate the handle relatively slower, and in some exemplary embodiments, resistance varies as the handle is rotated, providing a haptic feedback indicative of alignment of various components of the dispensing mechanism. Accordingly, manually operable dispensers as described herein have been found to improve dispensing performance by reducing occurrences of jamming and providing a more pleasing user experience.

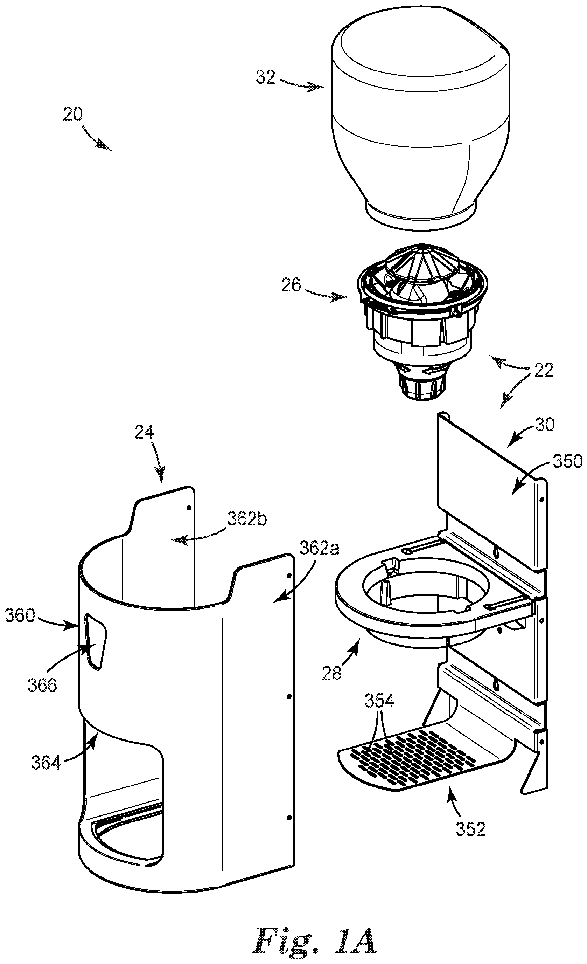

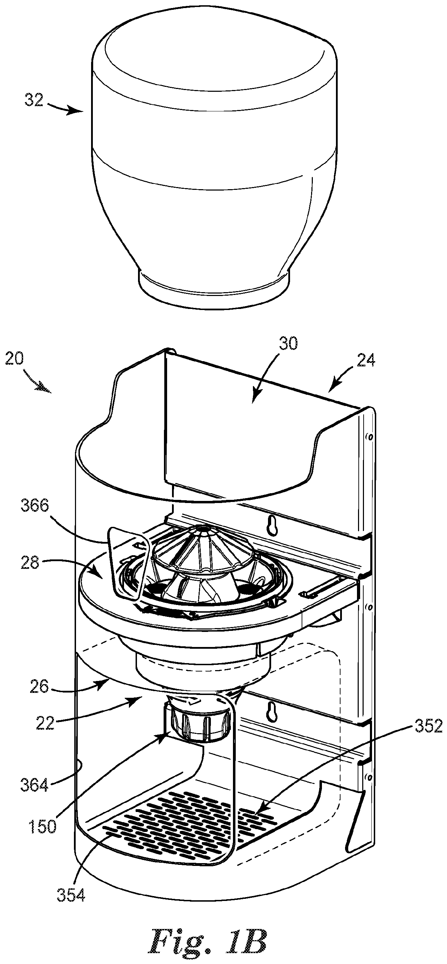

An exemplary embodiment of an earplug dispenser 20 is shown in FIGS. 1A and 1B. Dispenser 20 includes a dispensing unit 22 and an optional cover 24 (portions of which are illustrated as being transparent in FIGS. 1A and 1B). Dispensing unit 22, in turn, includes a dispensing mechanism 26 and a frame 28 maintained by an optional stand 30. Dispensing mechanism 26 is configured to interact with a container 32 containing a bulk supply of disposable earplugs (not shown), and in an exemplary embodiment is manually operable to individually dispense earplugs from the bulk supply. Frame 28 retains dispensing mechanism 26, with optional stand 30 supporting frame 28, and thus dispensing mechanism 26, relative to an installation surface (e.g., wall, table top, etc.). Where provided, cover 24 partially shields the individual earplugs from the surrounding environment as they are released from dispensing mechanism 26. Dispenser 20, and in particular dispensing mechanism 26, is configured to interface with and accurately dispense compressible, tacky surface earplugs with minimal occurrences of mechanism jamming.

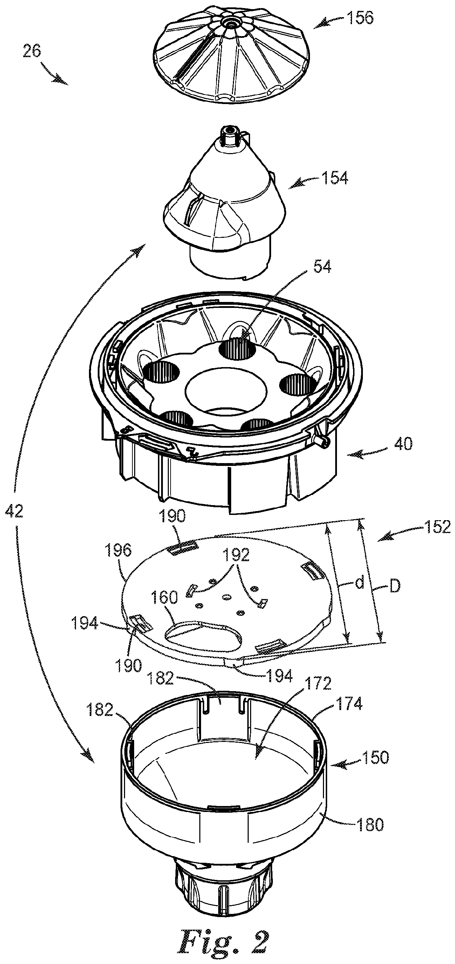

One exemplary embodiment of dispensing mechanism 26 is shown in greater detail in FIG. 2, and includes a separator assembly 40 and an index assembly 42 (referenced generally). Index assembly 42 includes a mixing body 154, plate 152, a handle 150, and a shield 156, and is rotatably coupled to separator assembly 40 such that index assembly 42 is rotatable relative to the separator assembly 40 about a longitudinal axis A. Several features optionally provided by the assemblies 40, 42 are described in further detail below with reference to longitudinal axis A.

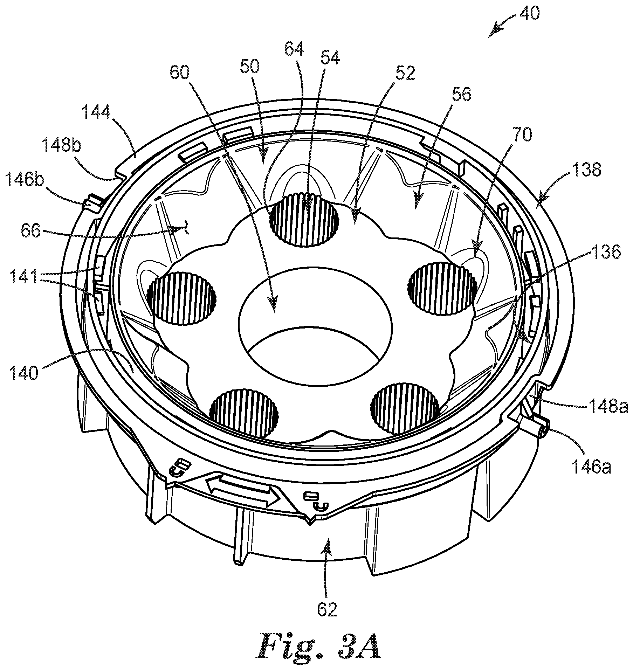

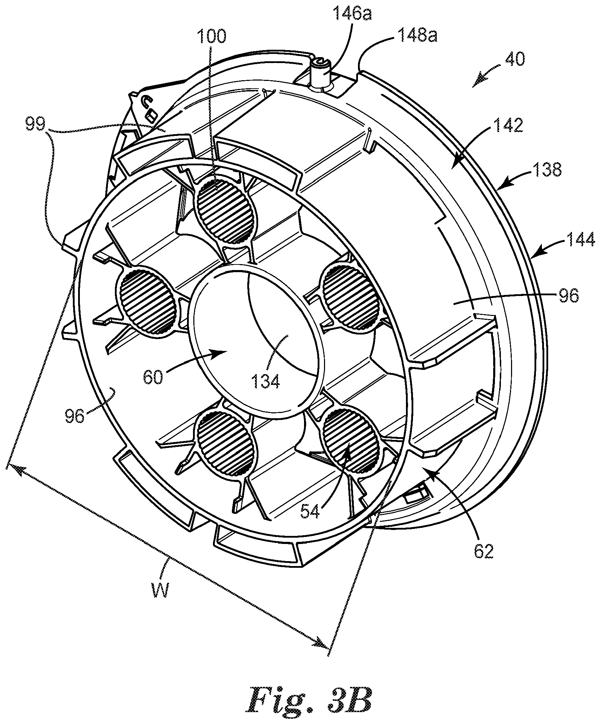

An exemplary embodiment of separator assembly 40 is shown in greater detail in FIGS. 3A and 3B, and includes or defines a guide wall 50, a platform 52, and a plurality of bores 54. Guide wall 50 extends from the platform 52, with the platform 52 and guide wall 50 combining to define a well 56 (referenced generally in FIG. 3A) within which earplugs (not shown) can accumulate. Bores 54 are each open to the well 56 at platform 52, and are configured to receive individual earplugs as described below. Separator assembly 40 further includes features that promote coupling with index assembly 42 (FIG. 2) and frame 28 (FIG. 1A), such as an inner hub 60 and an outer hub 62.

Guide wall 50 is generally configured to contain randomly arranged earplugs (not shown) on platform 52 and within well 56, and optionally incorporates one or more features that assist in directing individual earplugs into respective bores 54 and/or enhances mixing of the randomly arranged earplugs within well 56 during operation of the dispensing mechanism 26 (FIG. 2). As described in greater detail below, operation of the dispensing mechanism 26 entails rotating the index assembly 42 (FIG. 2) relative to the separator assembly 40 to release an individual earplug from respective bores 54. A new earplug (from the grouping of earplugs within well 56) then self-loads into the now-empty bore 54 via a corresponding entrance opening 64 in the platform 52. Guide wall 50 can assist in directing the individual earplug to the entrance opening 64 in a desired orientation. Further, index assembly 42 can include various features that interact with earplugs within well 56 upon rotation of index assembly 42, with this interaction desirably mixing, or reorienting, many of the earplugs within the grouping. Guide wall 50 optionally incorporates features that enhance the mixing effect caused by rotation of the index assembly 42.

Platform 52 can be a substantially planar body, extending between inner hub 60 and guide wall 50. Platform 52 serves to define a bottom or lower surface of well 56, as well as entrance opening 64 of each of bores 54. While the views reflect separator assembly 40 as providing five of bores 54, any other number, either greater or lesser, is equally acceptable. Bores 54 are arranged to extend in the longitudinal direction (e.g., a central axis of each of bores 54 is substantially parallel (e.g., within 5 degrees of a truly parallel relationship) with longitudinal axis A), from entrance opening 64 to an exit opening 100. Relative to the orientation of FIG. 3A, earplugs (not shown) are initially loaded into bores 54 from "above" platform 52, and are dispensed or released to a location "below" exit openings 100 (FIG 3B). In this regard, platform 52 can be viewed as defining an upper major face of separator assembly 40. In some embodiments, separator assembly 40 can have a generally hollow construction, with outer hub 62 defining a lower major face 102 of the separator assembly 40 opposite the platform 52. In other embodiments, separator assembly 40 can have a more solid construction and/or be integrally formed as a single component. Regardless, and as described in greater detail below, individual earplugs self-load into each of bores 54 at platform 52, followed by gravity-induced release of an individual earplug from corresponding bore 54 in a direction of lower major face 102.

Bores 54 can each have the cylindrical shape as shown. In other embodiments, the bores 54 can have a tapering shape, either increasing or decreasing in diameter in extension from the platform 52. In yet other embodiments, the bores 54 can be non-circular in transverse cross-section, for example having an oval-like perimeter shape.

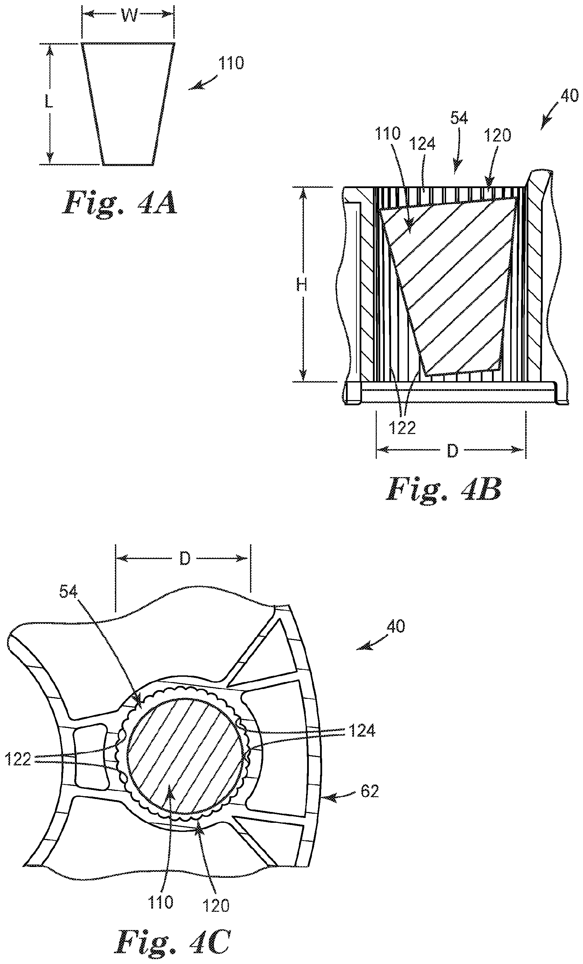

Bores 54 are generally sized and shaped to promote temporary retention of an individual earplug in an upright or lengthwise orientation. As a point of reference, disposable earplugs useful with the dispensers of the present disclosure can have a variety of different constructions (e.g., shapes, dimensions, materials, etc.), and bores 54 are configured to accurately interface or handle a number of differently configured earplug designs, styles or formats. In more general terms, however, and with reference to FIG. 4A, a disposable earplug 110 defines a length L and a maximum outer width (or diameter) W. The length L is conventionally greater than the maximum width W such that FIG. 4A depicts earplug 110 in an upright or lengthwise direction (i.e., a major axis defined by a shape of earplug 110 is arranged vertically). Disposable earplugs useful with the present disclosure can have a variety of different shapes, such as the conical-like shape shown in FIG. 4A, or other shapes such as cylindrical or cylindrical-like, or a more complex shape. The present disclosure is not limited to any particular disposable earplug shape or size. With this in mind, FIGS. 4B and 4C illustrate that each of bores 54 has a height H and minimum diameter D selected in accordance with the expected earplug length L and maximum width W, and in particular such that earplug 110 can only be completely received and arranged within bore 54 in the lengthwise direction. Bore diameter D is less than the expected length L of earplug 110 so that loaded earplug 110 occupies a majority of height H, thus preventing a second earplug (not shown) from completely loading into bore 54 "on top of' already-loaded earplug 110 (and thus only a single earplug 110 will subsequently be dispensed from bore 54). Stated otherwise, were bore 54 sized so that the earplug 110 could be arranged horizontally within bore 54 (or perpendicular to the height H of bore 54), a second earplug could undesirably also fully load within bore 54. However, bore diameter D is at least slightly greater than the expected earplug maximum width W to permit earplug 110 to readily enter or load within bore 54 in the lengthwise orientation. In this regard, in some embodiments the dispensers of the present disclosure are configured to be equally useful with a number of different earplug shapes and sizes (e.g., eleven different disposable earplug formats), with bore diameter D selected to be slightly greater than the largest earplug diameter from the earplug products intended to be used with the particular dispenser. The dimensional relationship between bore 54 and earplug 110 reflected in the views of FIGS. 4B and 4C (in which earplug 110 occupies a significant portion of bore height H and diameter D) is but one example. Dispensers and dispensing mechanisms of the present disclosure are equally useful with other earplug sizes, including those that are smaller than earplug 110 illustrated. Thus, other earplugs may be shorter (and thus occupy less of bore height H) and/or more narrow (and thus occupy less of bore diameter D) as compared to relative sizes of FIGS. 4B and 4C.

As identified in FIGS. 4B and 4C, each of bores 54 is generated or circumscribed by a wall surface 120. While in theory it may be possible for individual earplug 110 to reside within a corresponding one of bores 54 without contacting wall surface 120 of bore 54, in actual practice, earplug 110 will be in virtually constant contact with various regions of wall surface 120. With this in mind, in some embodiments wall surface 120 optionally incorporates one or more anti-bonding constructions or features that promote low friction interface with disposable earplug 110. More particularly, wall surface 120 is optionally configured to promote sliding interface with an outer surface of earplug 110 that may be at least somewhat tacky or sticky. The sliding interface can be provided by forming a macroscopic roughness at or on at least a portion of wall surface 120. For example, in some embodiments wall surface 120 forms or defines a plurality of longitudinal ribs 122. Ribs 122 collectively form a ribbed macrostructure, with circumferentially adjacent ribs 122 being separated by a groove 124. A radial height of each of ribs 122 (and thus a depth of each of the grooves 124) can be on the order of at least 0.3 mm, although other dimensions (either greater or lesser) are also acceptable. Ribs 122 can be uniformly formed about a circumference of wall surface 120, with a circumferential width of each of grooves 124 being on the order of not less than 1 mm although other dimensions (either greater or lesser) are also acceptable. Ribs 122 can generally follow the intended drop direction of earplug 110 (e.g., are substantially parallel (e.g., within 5% of a truly parallel relationship) with a center line of bore 54 and thus with longitudinal axis A). Alternatively, ribs 122 can be arranged at an angle relative to the bore center line, defining a slight spiral or twist or rifling in extension between opposing ends of wall surface 120. Finally, while ribs 122 have been illustrated as being formed or provided along an entirety of wall surface 120 (e.g., extending between platform 52 and an opposing, terminal end of bore 54), in other embodiments ribs 122 (or other earplug interface surface enhancing feature) can encompass only a portion of wall surface 120.

In other embodiments, ribs 122 are replaced by another form of macroscopic surface texturing or roughening (e.g., wall surface 120 can be knurled), or formed by a series of bumps or rings or other shaped protrusions configured to provide a low friction interface with a disposable earplug in contact therewith. In yet other embodiments, the optional anti-bonding construction or feature provided with wall surface 120 includes coating or forming wall surface 120 with a low surface energy material to reduce adhesive forces between earplug 110 and wall surface 120 (e.g., a low surface energy material is one that exhibits a tendency to repel, rather than attract, the sticky surface present on some disposable earplugs). The low surface energy material can be any material naturally exhibiting low surface energy or can be a material incorporated into a material of wall surface 120 (e.g., the separator assembly 40, and thus wall surface 120, can be a molded plastic, with the plastic material or resin including a low surface energy additive, such as a flouropolymer (e.g., available from 3M Company of St. Paul, MN under the trade name 3M™ Dyneon™)).

The optional anti-bonding constructions or features in accordance with the present disclosure include any surface feature that lessens frictional interface with an earplug as compared to the frictional interface that would otherwise be present between the earplug and a bore wall surface that did not include the anti-bonding construction or feature. The anti-bonding construction or feature can be chemical in nature (e.g., low surface energy material or coating), mechanical in nature (e.g., macroscopic roughness such as ribs), or a combination of both. In yet other embodiments, wall surface 120 can be smooth and formed of a material not having a low surface energy attribute.

In an exemplary embodiment, as best viewed in FIG. 3B, separator assembly 40 includes an outer hub 62 and an inner hub 60, optionally defining a passageway 134, that may receive and/or interact with one or more components of index assembly 42. Passageway 134 is co-axial with longitudinal axis A. In related embodiments, inner hub 60, and thus separator assembly 40, can be described as defining longitudinal axis A along the passageway 134.

Outer hub 62 includes an inner surface 96 and an outer surface 98. In an exemplary embodiment, outer hub 62 includes one or more stiffening elements 99 located around a perimeter of outer hub 62. Stiffening elements 99 provide selectively increased thickness and/or stiffness at particular locations of outer hub 62, and/or provide other functions, such as features to attach or retain separator assembly 40 with one or more other components of dispensing mechanism 26. That is, stiffening elements 99 may primarily serve to increase a stiffness of outer hub 62, and/or may provide additional utility.

Inner surface 96 of outer hub 62 generally forms a substantially annular shape that may exhibit a circular, oval, elliptical, or other suitable shape, and provides a cavity or space that a portion of index assembly 42, such as plate 152, may reside in. In an exemplary embodiment, outer hub 62 is sized to be slightly larger than a dimension of plate 152, and exhibits a suitable flexibility such that outer hub 62 may elastically bend or flex to accommodate plate 152, as described further herein. In various exemplary embodiments, outer hub 62 has a width (W) measured between opposite points on inner surface 96, along a line perpendicular to and passing through longitudinal axis A, between 50 mm and 300 mm, 75 mm and 200 mm, or of about 120 mm. Width (W) may be substantially uniform around a perimeter of inner surface 96, or may vary slightly due to a varying wall thickness, non-circular shape, elastic deformation when in use, or other variation.

Separator assembly 40 optionally includes or provides various features for receiving container 32 (FIG. 1A) and/or for mounting to frame 28 (FIG. 1A). In some embodiments, for example, separator assembly 40 includes or defines a capture ring 136 and a flange body 138. Capture ring 136 is coaxially disposed about outer hub 62 adjacent upper edge 68. A slot 140 is defined between capture ring 136 and outer hub 62, and is sized to receive a neck (not shown) of container 32 (FIG. 1A). In this regard, separator assembly 40 can include various features, such as locking tabs 141, which selectively capture the container neck within slot 140.

Returning to FIG. 2, dispensing mechanism 26 includes an index assembly 42 that facilitates mixing and/or funneling of earplugs towards an exit, and includes features that affect a rotational resistance felt by a user rotating handle 150. Plate 152 defines a dispensing aperture 160 and is connected to handle 150, mixing body 154 and shield 156. Handle 150 is rotatably coupled to separator assembly 40. Rotation of handle 150 about longitudinal axis A moves dispensing aperture 160 into alignment with respective individual bores 54 to release an earplug (not shown) from bore 54. Mixing body 154 and shield 156 may also rotate with handle 150, effectuating mixing of the grouping of earplugs within well 56.

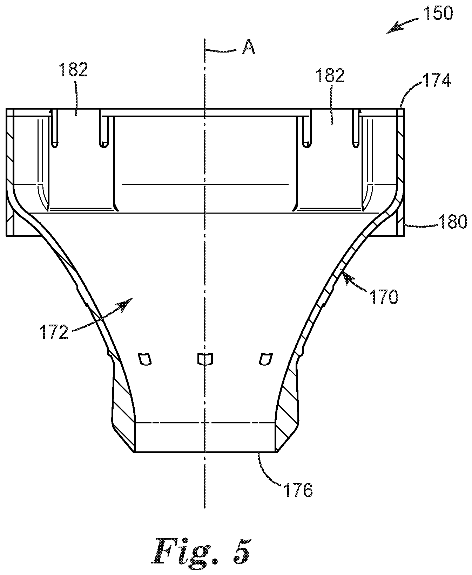

With additional reference to FIG. 5, handle 150 is a hollow body in some exemplary embodiments, and includes a side wall 170 defining a chamber 172. Handle 150 is open to chamber 172 at an upper end 174 and a dispensing end 176. Handle 150 can have various shapes and sizes, and in some exemplary configurations is symmetric about the longitudinal axis A (e.g., in some embodiments, handle 150 can be viewed as defining longitudinal axis A). Further, handle 150 can have a generally tapering diameter in a direction of dispensing end 176 (e.g., a funnel shape). As further described below, individual earplugs (not shown) are dispensed to a user from dispensing end 176, with the optional tapered or funnel shape of handle 150 establishing a reduced diameter at dispensing end 176 conducive to placement of a user's hand (and thus more accurate, funneled dispensing of an earplug into the user's palm). In various exemplary embodiments, handle 150 further includes features that promote rotation of handle 150 by a user's hand, such as finger grips 178.

Handle 150 includes or forms one or more features that facilitate assembly with complimentary features of plate 152. For example, side wall 170 forms a sleeve 180 at the upper end 174 and having an outer diameter commensurate with (e.g. slightly smaller than) an inner diameter of outer hub 62 of separator assembly 40. One or more tabs 182 are formed radially inward (relative to the longitudinal axis A) of sleeve 180 at upper end 174. Tab(s) 182 is configured for mounting with a corresponding feature of plate 152 as described below. A wide variety of other mounting configurations are also acceptable. For example, while in some embodiments handle 150 is indirectly coupled to separator assembly 40 via plate 152 (and mixing body 154), in other embodiments, handle 150 can be more directly rotatably mounted to separator assembly 40.

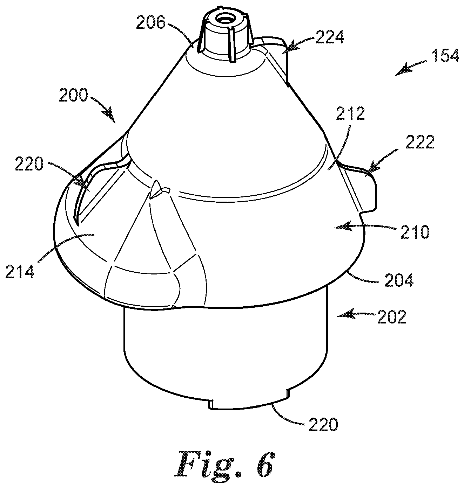

Mixing body 154 is shown in greater detail in FIG. 6, and includes or forms a mixing region 200 and a base 202. Mixing region 200 is sized and shaped to interface with earplugs (not shown) and other components of dispensing unit 22 (FIG. 1A) to promote flow of earplugs towards well 56 and ultimately to bores 54. Base 202 extends from mixing region 200, and is configured for mounted assembly with plate 152 (FIG. 2) and separator assembly 40 (FIG. 2).

Base 202 can assume various forms appropriate for mounting to separator assembly 40. In some embodiments, base 202 is cylindrically-shaped, sized to be rotationally received within passageway 134 of separator assembly 40. Base 202 can further be configured for coupling with plate 152 (FIG. 2). For example, base 202 forms one or more fingers 220 each sized to be frictionally received within a corresponding one of channels 192 (FIG. 2) in plate 152, as well as an interiorly threaded bore 232 for receiving a screw or other fastening member (that is also connected to plate 152). Other mounting configurations are equally acceptable. In yet other embodiments, base 202 can be configured for direct coupling to handle 150 (FIG. 2).

With reference to FIG. 2, plate 152 has a generally circular-shaped perimeter, and defines dispensing aperture 160. A size and shape of dispensing aperture 160 generally corresponds with a size (e.g., diameter) of exit opening 100 (FIG. 3B) of each of bores 54 (e.g., a cross-sectional area of dispensing aperture 160 is slightly larger than a cross-sectional area of each of exit openings 100). Further, a radial location (relative to longitudinal axis A of dispensing aperture 160 corresponds with a radial location (relative to longitudinal axis A) of each of bores 54 such that upon final assembly of dispensing unit 22 (FIG. 1A), dispensing aperture 160 can be longitudinally aligned with respective exit openings 100.

Plate 152 includes or forms various features that promote assembly with handle 150 in some embodiments. In an exemplary embodiment, plate 152 includes one or more slots 190 sized to receive corresponding tabs 182 associated with handle 150. Other mounting configurations are equally acceptable. In some embodiments of the present disclosure, handle 150 and plate 152 are configured to be rigidly coupled to one another (e.g., upon final assembly, plate 152 rotates with rotation of handle 150).

In addition, plate 152 optionally includes features configured for coupling with complimentary features provided with mixing body 154. For example, plate 152 can form one or more channels 192 configured for mounting with a corresponding feature of mixing body 154 as described below. A wide variety of other mounting configurations are also acceptable.

The present inventors have found that dispenser performance and user experience may be improved by controlling a force a user must exert to rotate handle 150 when operating dispenser mechanism 26. Certain predetermined force levels may prompt a user to operate the device at a relatively slower speed. This in turn reduces the likelihood of a compressible earplug being pinched or otherwise caught between components of separator assembly 40 and index assembly 42, for example, during movement. Furthermore, a force a user must exert may be selectively varied to provide a haptic feedback signaling alignment of various components of dispensing mechanism 26.

A torque required to rotate handle 150 (i.e. a rotational resistance of handle 150) may be affected in part by one or more dispensing mechanism portions of dispensing mechanism 26. In an exemplary embodiment, dispensing mechanism 26 includes a first dispensing mechanism portion and a second dispensing mechanism portion (e.g. all or a portion of frame 28, separator assembly 40, index assembly 42, etc.) that interact with each other during rotation of the handle. Interaction between the first dispensing mechanism portion and the second dispensing mechanism affects a rotational resistance (τ) at the handle such that rotational resistance (τ) varies between a maximum rotational resistance (τmax) and a minimum rotational resistance (τmin) as the handle is rotated by a user. In some exemplary embodiments, such interaction provides a varying resistance as handle 150 is rotated that may be perceived by a user as a signal of a relative position, and/or alignment, for example, of dispensing aperture 160 and respective exit openings 100 of bores 54. First and second dispensing mechanism portions may be any suitable portion of dispensing mechanism 26 that interact with one another during rotation of handle 150.

First dispensing mechanism portion and second dispensing mechanism portion may be provided by any suitable components of dispensing mechanism 26, and in an exemplary embodiment, at least first dispensing mechanism portion exhibits a varying stiffness due to structural features, material composition, or other suitable variation, for example. In this way, rotational resistance (τ) varies during rotation of handle 150 as second dispensing mechanism portion, for example, interacts with locations of first dispensing mechanism portion having varying stiffness, as described further herein.

In one exemplary embodiment shown in FIGS. 2-3B, dispensing mechanism includes a first dispensing mechanism portion of outer hub 62 and second dispensing mechanism portion of one or more lobes 194 that are positioned at, and/or protrude outwardly, from a peripheral edge 196 of plate 152, and may be integrally formed with plate 152. Lobes 194 are configured and sized to create a desired contact area between lobes 194 and inner hub 62, for example. Plate 152 has a minor diameter (d) at locations not including a lobe 194, and a major diameter (D) at a location including a lobe 194, measured along a radial line passing through a center of plate 152. In an exemplary embodiment, minor diameter (d) is between 50 mm and 300 mm, 75 mm and 200 mm, or about 200 mm, and major diameter (D) is between . 1 mm and 5 mm, .2 mm and 3 mm, or about .75 mm larger than minor diameter (d). In various exemplary embodiments, minor diameter (d) and major diameter (D) may vary at different locations of plate 152, e.g. due to respective lobes having different sizes or positioned opposite one another, for example. In an exemplary embodiment, minor diameter (d) is slightly smaller than width (W) of separator assembly 40 and major diameter (D) is slightly greater than width (W) of separator assembly 40, such that peripheral edge 196 of plate 152 only contacts outer hub 62 at lobes 194. Such dimensions provide desired contact and deformation of outer hub 62 without creating unduly large gaps between outer hub 62 and peripheral edge 196 of plate 152.

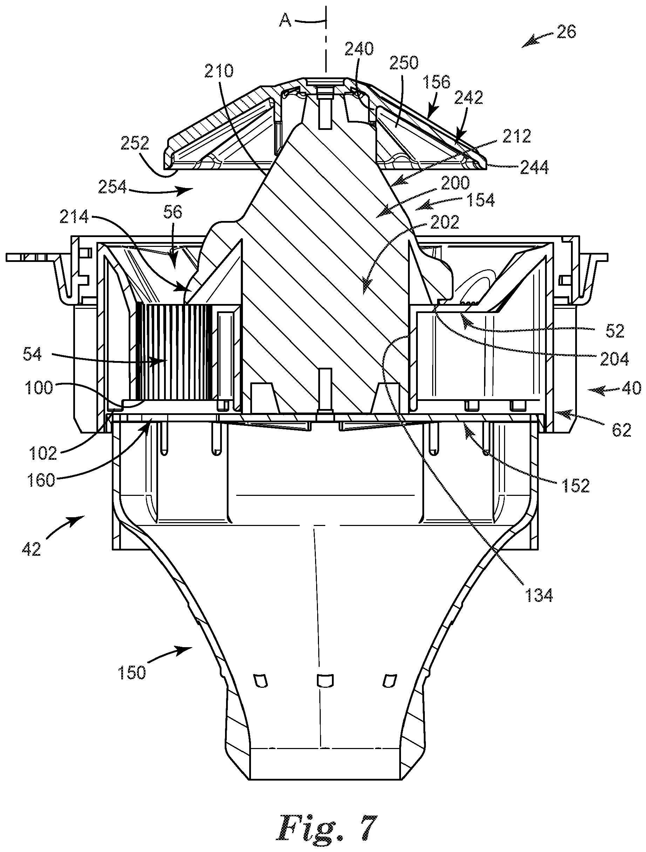

An exemplary final construction of dispensing mechanism 26 is shown in FIG. 7. Index assembly 42 is rotatably coupled to separator assembly 40 such that index assembly 42 can rotate relative to separator assembly 40 about longitudinal axis A. Base 202 of mixing body 154 is rotatably received within passageway 134 of separator assembly 40, locating mixing region 200 "above" (relative to the orientations of views) platform 52. Plate 152 is captured within outer hub 62 of separator assembly 40, at or slightly below lower major face 102. Lower end 204 of mixing region 200 is located within well 56 of the separator assembly 40, and dispensing aperture 160 can be brought into selective alignment with exit opening 100 of respective individual bores 54 with rotation of index assembly 42.

In an exemplary embodiment, plate 152 (and/or handle 150) is nested within outer hub 62 and configured to affect rotational resistance of index assembly 42. With plate 152 positioned within outer hub 62, first dispensing mechanism portion of outer hub 62 interacts with, and/or is in at least intermittent contact with, second dispensing mechanism provided by one or more lobes 194 during rotation. Contact between one or more lobes 194 results in frictional resistance and/or elastic deformation of outer hub 62 as index assembly 42 is rotated such that a restoring force acts against lobes 194 to return outer hub 62 to an original shape. In this way, a smooth, controllable force is created to affect a rotation resistance of handle 150 that must be overcome by a user to advance index assembly 142.

Second dispensing mechanism portion provided by lobes 194 are made of a durable material that may consistently slide along an appropriate surface without sticking or unduly damaging itself or the surface. In an exemplary embodiment, lobes 194 are made from a polyacetal or other suitable materials known in the art, and suitable combinations thereof.

First dispensing mechanism portion provided by outer hub 62 is made of a relatively flexible material that may elastically deform when acted on by lobes 194. In an exemplary embodiment, outer hub 62 is made from an acrylonitrile butadiene styrene (ABS), other suitable materials known in the art, and suitable combinations thereof.

A plate 154 and outer hub 62 constructed from different materials reduces the likelihood of squeaking or other undesirable interaction that may otherwise occur between the parts. Further, such materials can be selected to exhibit similar coefficients of thermal expansion and/or humidity, such that plate 154 and outer hub 62 behave similarly in different atmospheric conditions.

Outer hub 62 exhibits a thickness (t) that allows outer hub 62 to flex and elastically deform when acted on by lobes 194. In an exemplary embodiment, outer hub 62 exhibits a nonuniform wall thickness (t) that varies around the perimeter of outer hub 62, for example due to the presence of one or more stiffening elements 99 (FIG. 3B) positioned on outer hub 62. In various exemplary embodiments, thickness (t) is between 0.5 mm and 3 mm, 0.75 mm and 2 mm, or about 1 mm, particularly at locations where a stiffening element 99 is not present. In other exemplary embodiments, thickness (t) is substantially uniform around the perimeter of outer hub 62.

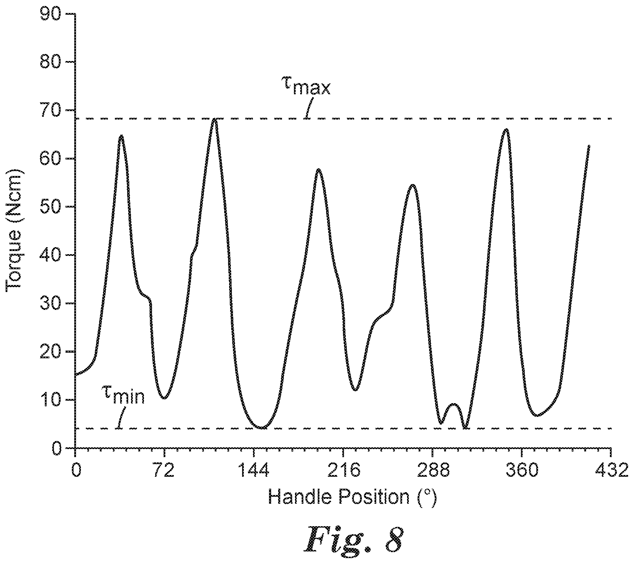

The structure of dispensing mechanism 26 may be configured as described herein to provide a haptic feedback signaling a relative position of dispensing aperture 160 and respective exit openings 100 of bores 54. For example, a torque a user must apply at handle 150 to overcome a rotational resistance may vary with relative positioning of separator assembly 40 and index assembly 42. FIG. 8 provides a representative graph of handle position (0° - 360°) versus rotational resistance at handle 150 (i.e. torque a user must apply to rotate handle 150). Rotational resistance varies between a maximum rotational resistance (τmax) and a minimum rotation resistance (τmin) as handle 150 is rotated between 0° and 360°. In an exemplary embodiment, handle 150 may be characterized by a first rotational resistance (τ1) at a first position (A), and a second rotational resistance (τ2) at a second position (B), different from first position (A). For example, position (A) may be a position at which dispensing aperture and an exit opening are not aligned and position (B) may be a position at which dispensing aperture and exit opening are sufficiently aligned to allow an earplug to be dispensed. In an exemplary embodiment, first rotational resistance (τ1) at position (A) is greater than second rotational resistance (τ2) at position (B). That is, first rotational resistance (τ1) is closer to a maximum rotational resistance (τmax) and second rotational resistance (τ2) is closer to a minimum rotational resistance (τmin). In this way, a user is prompted to stop rotating due to a reduced rotational resistance signaling alignment between dispensing aperture 160 and an exit opening 100.

In an exemplary embodiment, maximum rotational resistance (τmax) is sufficiently greater than minimum rotation resistance (τmin) such that a change in rotational resistance as handle 150 is rotated is readily perceived by a user. In an exemplary embodiment, maximum rotational resistance (τmax) is between 25 N·cm and 250 N·cm, 45 N·cm and 125 N·cm, 55 N·cm and 70 N·cm, or about 60 N·cm, and minimum rotation resistance (τmin) is between 0 N·cm and 50 N·cm, 2 N·cm to 25 N·cm, 5 N·cm to 10 N·cm, or about 7 N·cm. In various exemplary embodiments, maximum rotational resistance (τmax) is between 2 and 15, 5 and 12, or about 10 times minimum rotation resistance (τmin).

Dispenser mechanism 26 may be configured to provide any suitable toque profile as handle 150 rotates. In an exemplary embodiment, relatively heightened pressure between first dispensing mechanism portion, such as outer hub 62, and second dispensing mechanism portion, such as one or more lobes 194, occurs at or near locations of stiffening elements 99 due to, for example, a greater restoring force exerted by outer hub 62 at or near locations of stiffening elements 99. Accordingly, relative positioning of lobes 194, dispensing aperture 160, stiffening elements 99 and/or other components of dispensing mechanism 26 may be selected to result in a desired torque profile. In an exemplary embodiment represented by the torque profile shown in FIG. 8, lobes 194 and stiffening elements 99 are positioned such that a maximum rotational resistance (τmax) occurs between respective positions of alignment of dispensing aperture 160 and exit openings 100, and a relatively reduced rotational resistance occurs relatively nearer a position of alignment (e.g. 72°, 144°, 216°, etc.). In other exemplary embodiments, a maximum rotational resistance (τmax) may occur near or at a position where dispensing aperture and an exit opening 100 are aligned while a reduced rotational resistance occurs where dispensing aperture and an exit opening 100 are not aligned. Similarly, in various exemplary embodiments, a rotational resistance may step or quickly transition from heightened and lessened values, rather than transition smoothly from between first and second positions. In various exemplary embodiments, a torque profile is similar regardless of the direction handle 150 is rotated. That is, a slope of the torque profile is similar when handle 150 is rotated in either direction from a location of maximum rotational resistance (τmax) such that a user will receive the same or similar haptic feedback.

Desirable torque profiles may be provided by any suitable first and second dispensing mechanism portions. In various exemplary embodiments, the representative torque profile of FIG. 8, or other torque profile that may be desired for a particular application, may be achieved with first and second dispensing mechanism portions including, respectively, an annular hub, such as inner hub 60 and/or outer hub 62, plate 152, a major surface and/or peripheral edge of plate 152, one or more lobes such as lobes 194, any suitable surface and/or component of dispensing mechanism 26, and suitable combinations thereof, as selected by one of skill in the art, at least one of which is configured to exhibit a varying stiffness and that can interact with the other during rotation of handle 150. In an exemplary embodiment, an annular hub having an oval or elliptical shape interacts with a plate 152 having an oval or elliptical plate. As the annular hub and plate rotate relative to each other, for example about a longitudinal axis, pressure between the two components varies and may be configured to result in a desired torque profile at handle 150.

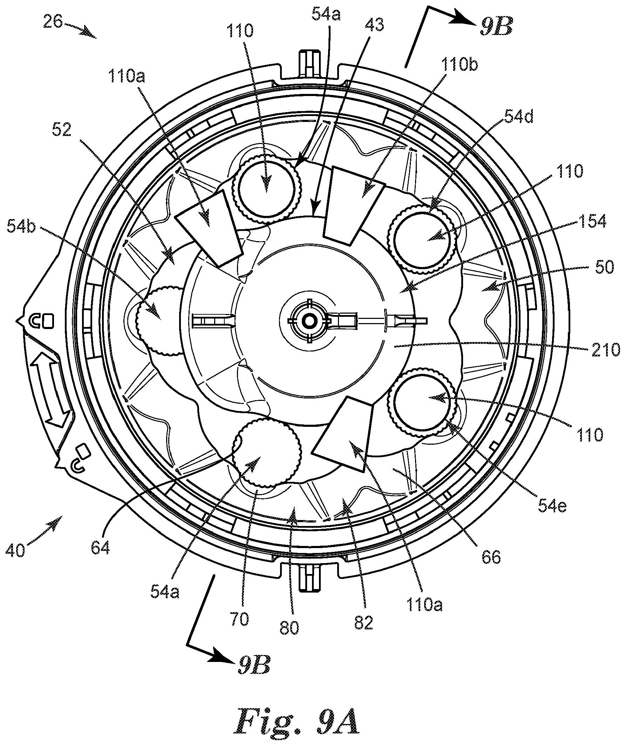

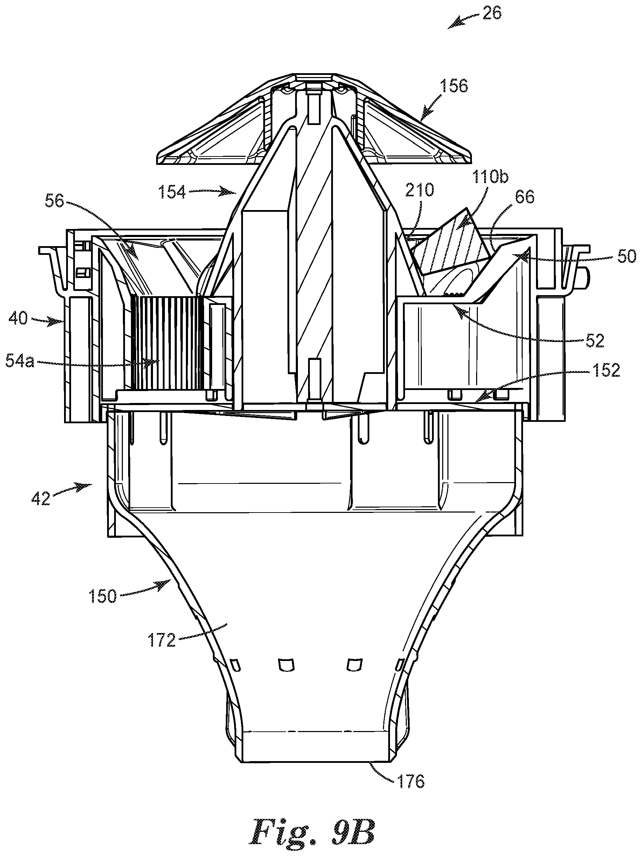

Operation of dispensing mechanism 26 in handling disposable earplugs 110 for subsequent dispensing is generally reflected in FIGS. 9A through 9D. For ease of illustration and understanding, optional shield 156 is removed from view in FIG. 9A. In the operational state of FIG. 9A, first and second bores 54a, 54b are empty, whereas each of the third through fifth bores 54c-54e are loaded with an earplug 110 (for example, the arrangement of FIG. 9A can be representative of a scenario in which an earplug has just been dispensed from the second bore 54b). Several additional earplugs 110a-110c are loosely or randomly arranged within well 56 in a vicinity of platform 52. It will be understood that un-loaded earplugs will naturally and randomly assume virtually any orientation, and any un-loaded earplugs proximate well 56 will randomly contact dispensing mechanism 26 at any available surface such that arrangements of FIG. 9A are merely one example. In some embodiments, a spacing between and geometry of mixing body 154 and guide wall 50 encourages at least some of un-loaded earplugs 110a-110c slightly away from platform 52 and into an orientation conducive to subsequent self-loading into an open bore 54. For example, FIG. 9B illustrates one possible, naturally occurring orientation of second un-loaded earplug 110b. With cross-reference between FIGS. 9A and 9B, a radial distance between guide face 66 of guide wall 50 and exterior surface 210 of mixing body 154 is less than a length of earplug 110b such that when arranged in orientation of FIGS. 9A and 9B, earplug 110b is lifted slightly above platform 52. Further, opposing taper angles of guide wall 50 and mixing body 154 tilts earplug 110b (i.e., a centerline of earplug 110b is non-parallel with the plane of platform 52), with this tilted orientation being conducive to earplug 110b self-loading within an open one of bores 54 once aligned as described below. It will be understood that the location and orientation of second earplug 110b in FIGS. 9A and 9B is only one possibility, and in many instances, un-loaded earplugs can and will be in contact with platform 52. Further, other disposable earplugs useful with the present disclosure can have a shorter length and thus may not span across guide wall 50 and mixing body 154 even in orientation of FIG. 9B. By optionally lifting at least some of un-loaded earplugs 110a-110c away from platform 52, the likelihood of a stray, un-loaded earplug becoming lodged within gaps between moving parts of dispensing mechanism 26 is reduced, thus minimizing malfunctions or "jamming" of dispensing mechanism 26.

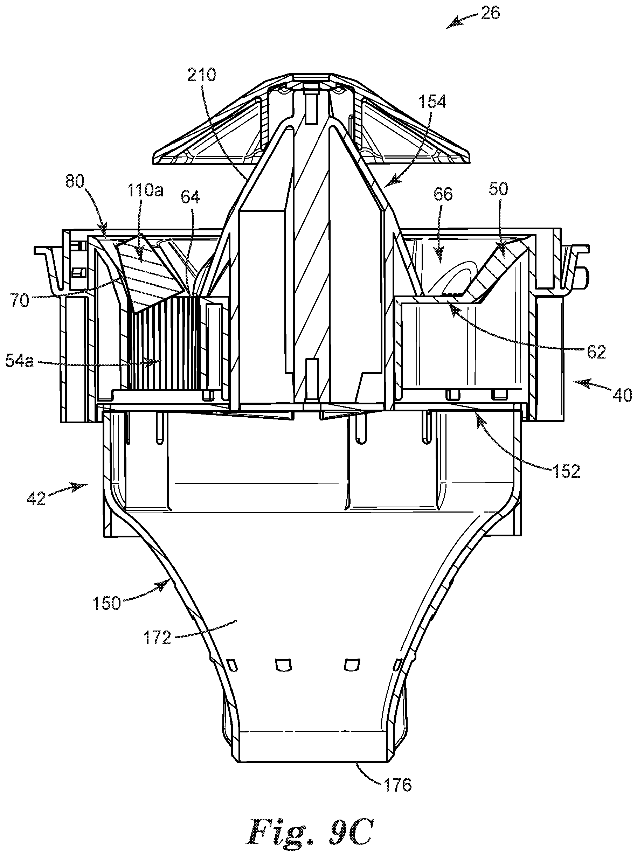

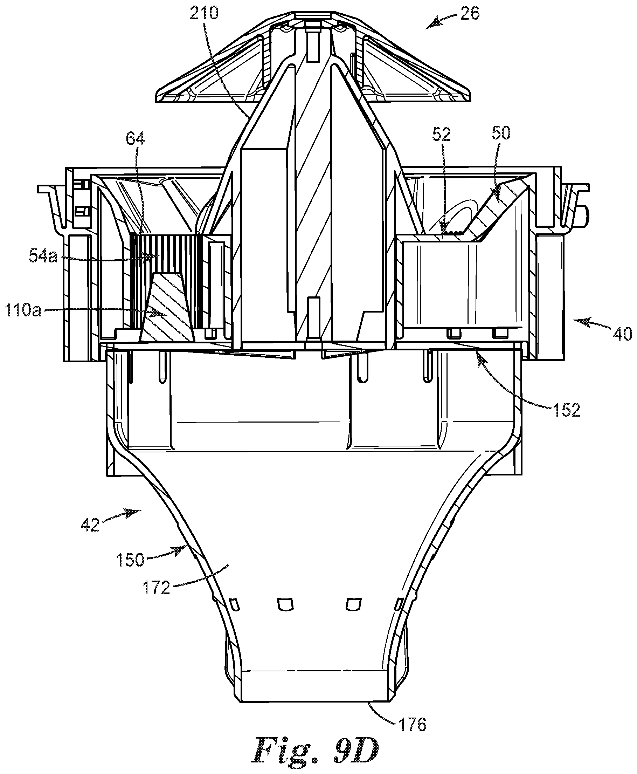

With rotation of index assembly 42 (e.g., by user-applied rotational force at handle 150) relative to separator assembly 40 (e.g., clockwise relative to orientation of FIG. 9A) and/or due to gravity, first un-loaded earplug 110a becomes aligned with and self-loads into first bore 54a. For example, rotation of mixing body 154 directly causes the first un-loaded earplug 110a to move toward first bore 54a and/or the mixing body 154 is in contact with other un-loaded earplugs within the well 56 and rotation of mixing body 154 causes an entire grouping of un-loaded earplugs, including first un-loaded earplug 110a, to move toward first bore 54a. As the first un-loaded earplug 110a is caused to slide, roll, or otherwise articulate along guide face 66, the first un-loaded earplug 110a progresses from channel region 82 to ramp region 80 associated with first bore 54a. Once in channel region 80, the first un-loaded earplug 110a interfaces with corresponding trough 70 as shown in FIGS. 9C and 9D. In this regard, a radial distance between guide face 66 (along the trough 70) and exterior surface 210 of the mixing body 154 tapers in a direction of platform 52, allowing earplug 110a to drop (due to gravity) toward the entrance opening 64 of first bore 54a. Further, a shape of trough 70 assists in guiding earplug 110a to slide directly into bore 54a in an upright or lengthwise manner. Trough 70 encourages individual earplug 110a to readily drop into open bore 54a, and as earplug 110a drops or slides along either trough 70 or exterior surface 210 of mixing body 154 (or both), earplug 110a is naturally oriented lengthwise. Once inside bore 54a (FIG. 9D), earplug 110a can rest on plate 152, sliding along a surface of plate 152 as index assembly 42 is further rotated.

Once index assembly 42 has been rotated relative to separator assembly 40 so as to align dispensing aperture 160 with exit opening 100 of a respective bore, earplug 110a is released and falls through dispensing aperture 160 due to gravity. The so-released earplug 110 drops through chamber 172 of handle 150 and is dispensed into a hand of the user at dispensing end 176. With further, continued rotation of index assembly 42, a new earplug 110 will self-load into the now-open bore as described above for subsequent dispensing.

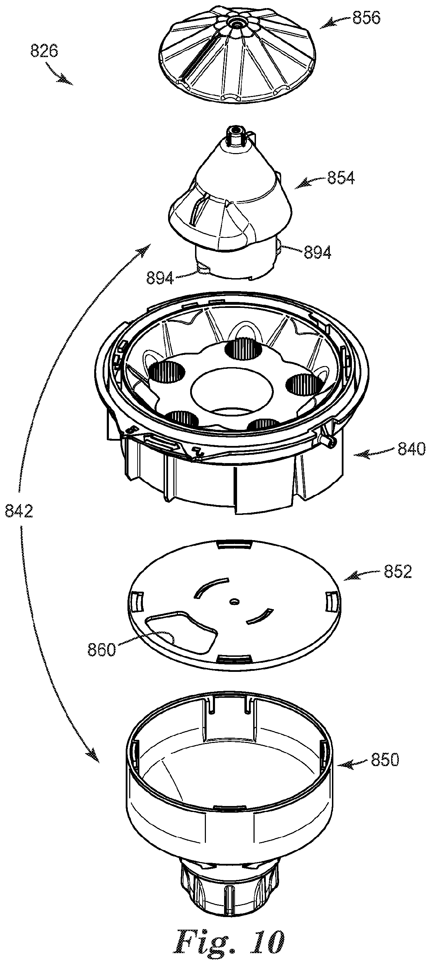

FIG. 10 shows another exemplary embodiment of a dispensing mechanism 826 including a separator assembly 842 and an index assembly 842. Similar to that of dispensing mechanism 26 described herein, index assembly 842 includes a plate 852 defining a dispensing aperture 860 and that may be connected to handle 850, mixing body 854 and shield 856, and includes first and second dispensing mechanism portions that interact to affect a rotational resistance resistance (τ) at the handle such that rotational resistance (τ) varies between a maximum rotational resistance (τmax) and a minimum rotational resistance (τmin) as the handle is rotated by a user, as described above. In an exemplary embodiment, separator assembly 842 includes a first dispensing mechanism portion at inner hub 860 and second dispensing mechanism portion at one or more lobes 894 positioned on mixing body 854. In use, one or more lobes 894 interact with and/or are in at least intermittent contact with inner hub 860 to provide a haptic feedback to a user rotating handle 150. As described above with reference to dispensing mechanism 26, interaction of lobes 894 with a surface of inner hub 860, and/or deformation of inner hub 860 due to contact with lobes 894, affects a rotational resistance that a user must overcome to advance handle 860. The geometry, material composition, surface properties, positioning of one or more stiffening elements, and other characteristics of lobes 894 and outer hub 862 may be configured to provide a desired, predetermined rotational resistance at specified positions.

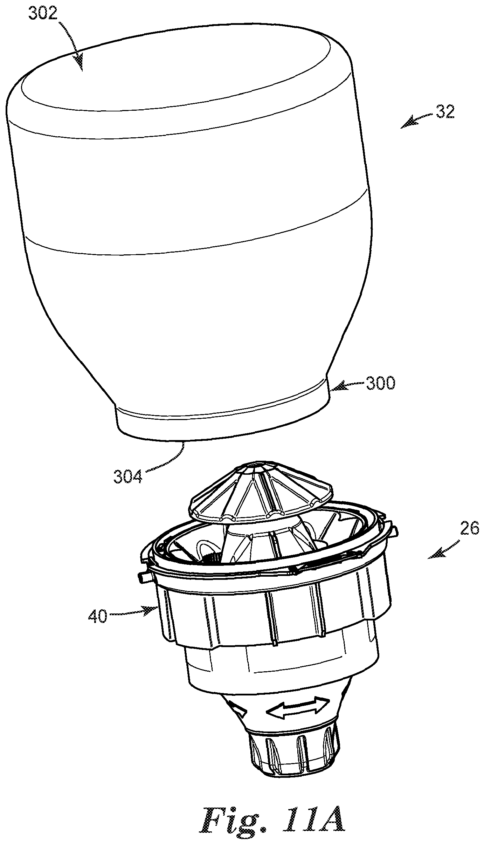

While FIGS. 9A through 9D reflect operation of dispensing mechanism 26 relative to a few earplugs, it will be understood that the dispensing mechanisms of the present disclosure are useful in handling and dispensing individual earplugs from a bulk supply. For example, separator assembly 40 is configured for selective assembly to a container of earplugs. FIG. 11A illustrates one embodiment of container 32 relative to dispensing mechanism 26. Container 32 can assume a wide variety of forms, and can be sized to contain any number of earplugs (not shown). Thus, the present disclosure is in no way limited to container 32 as shown. In general terms, container 32 provides an enclosed volume within which the supply of earplugs is retained. Container 32 forms a neck 300 opposite a floor 302. Neck 300 terminates at an open end 304 (referenced generally) that is open to the internal volume. As a point of reference, a cover (not shown) can be provided with container 32 for temporarily closing open end 304. Thus, for example, prior to mounting to dispensing mechanism 26, container 32 can be closed and stored in an upright orientation via floor 302. Regardless, a size and shape of neck 300 corresponds with geometric features provided with separator assembly 40 in a manner promoting releasable mounting of container 32 to dispensing mechanism 26.

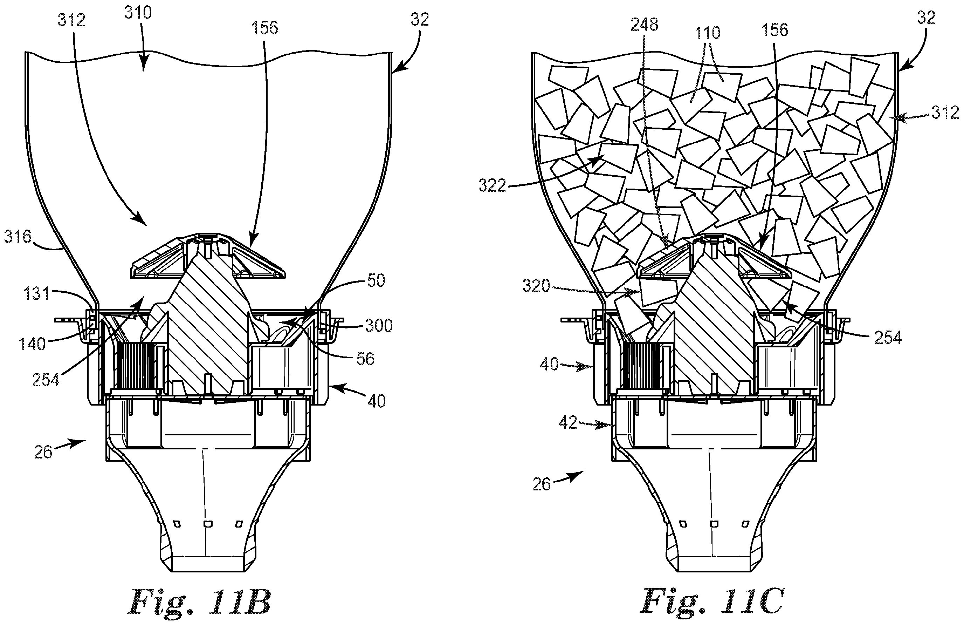

More particularly, and with reference to FIG. 11B, releasable assembly of container 32 to dispensing mechanism 26 includes insertion of neck 300 into slot 140 provided with separator assembly 40. A more robust connection between container 32 and dispensing mechanism 26 can be achieved via optional locking tabs 141 (FIG. 3A) and/or other components. Regardless, a size and shape of shield 156 is such that neck 300 is easily introduced over shield 156 and into engagement with separator assembly 40. In some embodiments, a size and shape of neck 300 corresponds with a shape and spatial location of guide wall 50 such that upon final assembly, a tapering region 306 of container 32 is generally aligned with the angular orientation of guide wall 50 such that earplugs (not shown) within container 32 naturally flow toward and along guide wall 50.

Upon assembly of container 32 to dispensing mechanism 26, an effective storage volume 310 is collectively defined by container 32 and dispensing mechanism 26. Effective storage volume 310 includes an open volume of container 32 and well 56 of dispensing mechanism 26. With this in mind, shield 156, where provided, divides effective storage volume 310 into two chambers. First chamber 254, as described above, is established between shield 156 and platform 52. A second chamber 312 is established above shield 156 (relative to orientation of FIG. 11B). When effective storage volume 310 is relatively full of disposable earplugs 110 as shown in FIG. 11C, a first grouping 320 (referenced generally) of earplugs 110 will naturally reside or accumulate within first chamber 254, and a second grouping 322 (referenced generally) of earplugs 110 will naturally reside or accumulate within second chamber 312. That is, due to gravity, some of earplugs 110 initially within just container 32 will fall into first chamber 254 as container 32 is mounted onto dispensing mechanism 26 (or, with an alternative mounting technique in which container 32 is oriented with neck 300 facing upwards and dispensing mechanism 26 is placed on to neck 300, some of earplugs 110 within container 32 will drop into first chamber 254 as assembled dispensing mechanism 26/container 32 is then rotated to orientation of FIG. 11C).

As individual earplugs 110 of first grouping 320 are incrementally dispensed from first chamber 254 with operation of dispensing mechanism 26 (as described above), various earplugs 110 of second grouping 322 will naturally move from second chamber 312 into first chamber 254 due to gravity. However, shield 156 effectively prevents a collective weight of second grouping 322 from acting upon first grouping 320 within first chamber 254. As a result, earplugs 110 within first chamber 254 are more loosely maintained relative to one another, and thus can more easily be separated from one another (with rotation of the index assembly 42) and become guided or loaded into individual bores 54 (FIG. 3A) as described above.

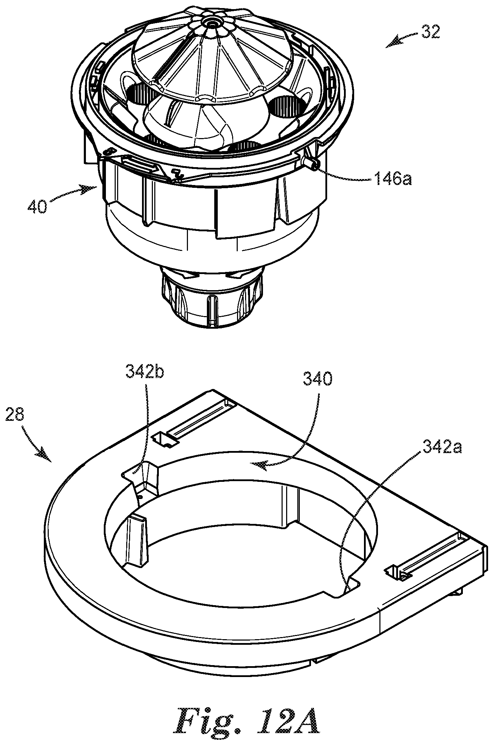

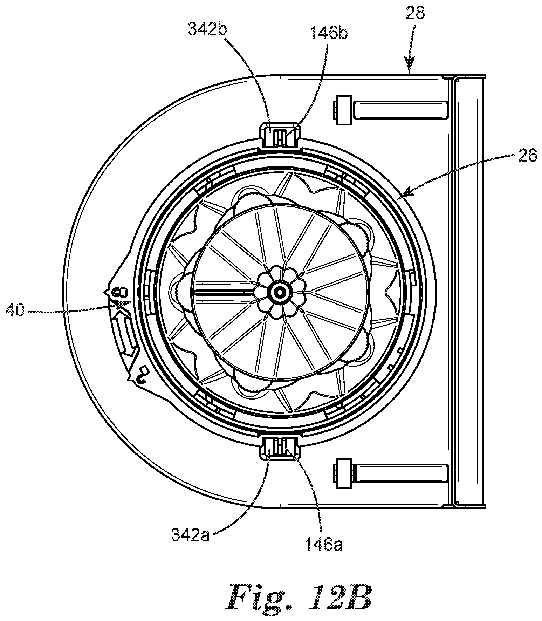

As indicated above, manual operation of dispensing mechanism 26 generally entails user-caused rotation of index assembly 42 relative to separator assembly 40 (and thus relative to bores 54). With this in mind, dispensers of the present disclosure can include components that spatially retain separator assembly 40 at a desired location and in a manner that spatially "holds" the separator assembly 40 during rotation of index assembly 42. For example, FIG. 1A illustrates frame 28 provided as part of stand 30. As shown in FIG. 12A, frame 28 forms a passage 340 that is sized and shaped to receive separator assembly 40. In this regard, frame 28 is optionally configured, in tandem with separator assembly 40, such that separator assembly 40 (and thus dispensing mechanism 26) can be removably mounted to passage 340. Moreover, frame 28 and separator assembly 40 incorporate complimentary features that fix separator assembly 40 to frame 28 such that separator assembly 40 cannot rotate relative to frame 28. For example, frame 28 can form opposing cavities 342a, 342b sized and shaped to receive a respective one of pins 146a, 146b (one of which is visible in FIG. 12A) provided with separator assembly 40. With additional reference to FIG. 12B, upon placement of pins 146a, 146b within corresponding cavity 342a, 342b, separator assembly 40 is thus supported by frame 28, and cannot freely rotate relative to frame 28. In some embodiments, clips (not shown) can be assembled to each of pins 146a, 146b, respectively, and are configured to achieve a more robust, press fit-type coupling between pins 146a, 146b and frame 28. Alternatively, a wide variety of other mounting constructions are equally acceptable.

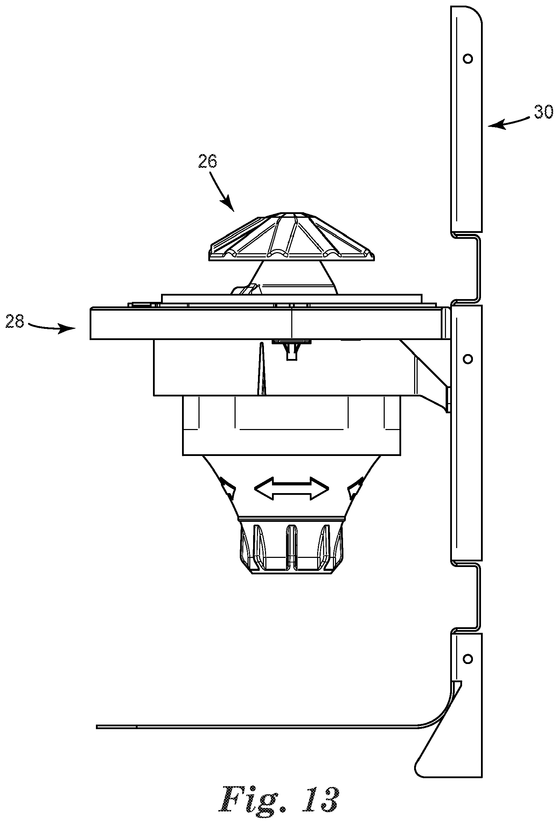

In some embodiments, frame 28 can be directly assembled to a surface of interest (e.g., a vertical wall). In other embodiments, frame 28 can be provided as part of stand 30 that otherwise incorporates additional, optional structures that serve to support frame 28 as shown in FIG. 1A. For example, stand 30 can include or form a back wall 350 and a bottom wall 352. Frame 28 is coupled to back wall 350 and arranged such that bottom wall 352 projects underneath frame 28. In some embodiments, back wall 350 can incorporate various features that promote assembly to a vertical surface (e.g., a wall). Where provided, bottom wall 352 serves as a catch for earplugs (not shown) released from dispensing mechanism 26, and can include or form water drainage holes 354. In other configurations, bottom wall 352 can be omitted. Final mounting of dispensing mechanism 26 to stand 30 is shown in FIG. 13.

With reference to FIGS. 1A and 1B, dispensing unit 22 can optionally be further protected from the environment by cover 24. Cover 24 includes or defines a front panel 360 and opposing side panels 362a, 362b. Front panel 360 forms an access opening 364 and a fill level opening 366. Side panel 362a, 362b are sized and shaped for assembly to back wall 350 of stand 30, with access opening 364 being sized and shaped to facilitate insertion of a user's hand. Fill level opening 366 is located to promote viewing of components within cover 24 as described below.

For example, and with specific reference to FIG. 1B, a user can obtain individual earplugs (not shown) by inserting his or her hand through access opening 364 and grasping handle 150. The user then rotates handle 150 to cause a single earplug to be dispensed into the user's hand as described above. Notably, cover 24 serves to protect the so-dispensed earplug from the surrounding environment (e.g., wind, rain, etc.). Moreover, drainage holes 354 in bottom wall 352 allow any water (e.g., rain) entering access opening 364 to readily drain away. Thus, dispenser 20 is highly amenable for installation at a plethora of different locations, including outdoor use. Finally, fill level opening 366 is generally aligned with a portion of container 32 (as assembled to dispensing mechanism 26), thus allowing a user to visually estimate the quantity of earplugs remaining within container 32.

The disposable earplug dispensing mechanisms and related earplug dispensers of the present disclosure provide a marked improvement over previous designs. The dispensing mechanism is easy to manually operate, and accurately dispenses earplugs from a bulk supply on an individual basis with minimal occurrences of jamming. A dispenser providing a haptic feedback to a user may prompt a user to rotate the handle at an appropriate speed to minimize occurrences of jamming and/or to signal alignment of various components such that the user may be made aware how far to rotate the handle in order to dispense a signal earplug. A user may thus react to stop movement of the handle to provide time for an earplug to be dispensed by force of gravity. These features and advantages are accomplished by a dispensing mechanism as described herein that is easily manufacturable and with minimum moving parts that could malfunction or wear out.

The foregoing detailed description and examples have been given for clarity of understanding only. No unnecessary limitations are to be understood there from. It will be apparent to those skilled in the art that many changes can be made in the embodiments described without departing from the scope of the disclosure. Any feature or characteristic described with respect to any of the above embodiments can be incorporated individually or in combination with any other feature or characteristic, and are presented in the above order and combinations for clarity only. Thus, the scope of the present disclosure should not be limited to the exact details and structures described herein, but rather by the structures described by the language of the claims, and the equivalents of those structures.

### Examples

The characteristics, operation, and advantages of the present invention will be further described with regard to the following detailed non-limiting examples. These examples are offered to further illustrate the various specific and preferred embodiments and techniques. It should be understood, however that many variations and modifications may be made while remaining within the scope of the present invention.

### Procedure 1: Rotational Resistance Test

Rotational resistance may be measured as a function of position of the handle of a dispenser mechanism. Two KEVLAR cords were wound approximately two revolutions around a handle modified by removing ribs or grip features as necessary to provide a consistent surface for the cords to contact. An end of one cord was fixed to the top clamp, and an end of the other cord fixed to the bottom clamp, of a Z020 Tensile Tester having a 200N load cell available from Zwick Roell. The dispenser mechanism is manually held in a horizontal orientation to avoid rotation while allowing vertical movement while the clamps were moved apart. Torque was calculated from the measured pull force and the handle radius. No container or earplugs were present.

**Example 1:** A dispensing mechanism as shown in FIG. 2 and including a plate having a minor diameter (d) of 119.2 mm, major diameter (D) of 119.95 mm (radius of 60.35 mm at lobe location), and a separator assembly having an outer hub with a width (W) of 120.5 mm (radius of 60.25 mm) were provided. Five bores of the separator assembly were evenly positioned at approximately 72° intervals. The plate was made from CELECON M90 polyacetal available from Celanese Corporation and the separator assembly was made from POLIMAXX GA300 available from IRPC Public Company Limited. The assembly was tested according to Procedure 1: Rotational Resistance Test, and the data shown in Table 1 was obtained.

**Table 1**

| **Position** | **Resistance (N·cm)** |
|---|---|
| 0° | 76.0 |
| 18° | 23.9 |
| 36° | 15.8 |
| 54° | 30.5 |
| 72° | 57.7 |
| 90° | 31.2 |
| 108° | 13.6 |
| 126° | 42.0 |
| 144° | 61.9 |
| 162° | 10.8 |
| 180° | 6.1 |
| 198° | 21.0 |
| 216° | 47.8 |
| 234° | 39.6 |
| 252° | 11.6 |
| 270° | 26.5 |
| 288° | 48.9 |
| 306° | 32.4 |
| 324° | 9.1 |
| 342° | 10.0 |
| 360° | 52.7 |

The dispensing mechanism of Example 1 demonstrated a varying rotational resistance at the handle that provided a haptic feedback to a user. Force was relatively higher at positions of which dispensing aperture and an exit opening were not aligned, and relatively lower at positions of which dispensing aperture and an exit opening were aligned such that an individual earplug could be dispensed (i.e. approximately 36°, 108°, 180°, 252°, 324°).

## Claims

1. A manually operable dispenser for dispensing earplugs, comprising:
a dispensing mechanism defining a longitudinal axis and comprising,
a separator assembly defining a well and one or more bores each open to the well;
an index assembly including a handle and a plate connected to the handle defining a dispensing aperture, the index assembly rotatably coupled with the separator assembly such that individual bores may be selectively aligned with the dispensing aperture by user rotation of the handle;
a first dispensing mechanism portion; and
a second dispensing mechanism portion that interacts with the first dispensing mechanism portion during rotation of the handle;
wherein interaction between the first dispensing mechanism portion and the second dispensing mechanism affects a rotational resistance (τ) at the handle such that rotational resistance (τ) transitions between a maximum rotational resistance (τmax) and a minimum rotational resistance (τmin) as the handle is rotated by a user, and wherein the rotational resistance indicates an alignment position of the dispensing aperture with respect to an exit opening of the one or more bores of the manually operable dispenser.

2. The dispenser of claim 1, wherein the first dispensing mechanism portion has a varying stiffness.

3. The dispenser of claim 1, wherein the dispensing mechanism exhibits a first rotational resistance (τ1) at the handle when the dispensing aperture is aligned with one of the plurality of bores, and a second rotational resistance (τ2) at the handle when the dispensing aperture is not aligned with one of the plurality of bores, and (τ2) > (2^{∗}τ1).

4. The dispenser of claim 1, wherein 25 N·cm < (τmax) < 150 N·cm.

5. The dispenser of claim 1, wherein 0 N·cm < (τmin) < 25 N·cm.

6. The dispenser of claim 1, wherein 45 N·cm < (τmax) < 85 N·cm and 2 N·cm < (τmin) < 15 N·cm.

## Patentansprüche

1. Eine manuell betätigbare Abgabevorrichtung zum Abgeben von Ohrstöpseln, umfassend:
einen Abgabemechanismus, der eine Längsachse definiert und Folgendes umfasst,
eine Separatorbaugruppe, die eine Vertiefung und eine oder mehrere Bohrungen definiert, die jeweils zu der Vertiefung offen sind;
eine Indexbaugruppe mit einem Handgriff und einer Platte, die mit dem Handgriff verbunden ist und eine Abgabeapertur definiert, wobei die Indexbaugruppe drehbar mit der Separatorbaugruppe verbunden ist, so dass einzelne Bohrungen wahlweise mit der Abgabeapertur durch Benutzerdrehung des Handgriffs ausgerichtet werden können;
einen ersten Abgabemechanismusabschnitt; und
einen zweiten Abgabemechanismusabschnitt, der mit dem ersten Abgabemechanismusabschnitt während der Drehung des Handgriffs zusammenwirkt;
wobei die Wechselwirkung zwischen dem ersten Abgabemechanismusabschnitt und dem zweiten Abgabemechanismus einen Drehwiderstand (τ) am Handgriff derart beeinflusst, dass der Drehwiderstand (τ) zwischen einem maximalen Drehwiderstand (τmax) und einem minimalen Drehwiderstand (τmin) übergeht, wenn der Handgriff von einem Benutzer gedreht wird, und wobei der Drehwiderstand eine Ausrichtungsposition der Abgabeapertur in Bezug auf eine Ausgangsöffnung der einen oder mehreren Bohrungen der manuell betätigbaren Abgabevorrichtung anzeigt.

2. Die Abgabevorrichtung nach Anspruch 1, wobei der erste Abgabemechanismusabschnitt eine veränderliche Steifigkeit aufweist.

3. Die Abgabevorrichtung nach Anspruch 1, wobei der Abgabemechanismus einen ersten Drehwiderstand (τ1) an dem Handgriff aufweist, wenn die Abgabeapertur mit einer der Vielzahl von Bohrungen ausgerichtet ist, und einen zweiten Drehwiderstand (τ2) an dem Handgriff, wenn die Abgabeapertur nicht mit einer der Vielzahl von Bohrungen ausgerichtet ist, und (τ2) > (2^{∗}τ1) ist.

4. Die Abgabevorrichtung nach Anspruch 1, wobei 25 N·cm < (τmax) < 150 N·cm.

5. Die Abgabevorrichtung nach Anspruch 1, wobei 0 N·cm < (τmin) < 25 N·cm.

6. Die Abgabevorrichtung nach Anspruch 1, wobei 45 N·cm < (τmax) < 85 N·cm und 2 N·cm < (τmin) < 15 N·cm.

## Revendications

1. Distributeur opérationnel manuellement pour distribuer des bouchons d'oreille, comprenant :
un mécanisme de distribution définissant un axe longitudinal et comprenant,
un ensemble séparateur définissant un puits et un ou plusieurs trous chacun ouvert sur le puits ;
un ensemble d'index incluant une poignée et une plaque reliée à la poignée définissant une ouverture de distribution, l'ensemble d'index couplé de manière rotative à l'ensemble séparateur de telle sorte que des trous individuels peuvent être alignés sélectivement avec l'ouverture de distribution par rotation par l'utilisateur de la poignée ;
une première partie de mécanisme de distribution ; et
une deuxième partie de mécanisme de distribution qui interagit avec la première partie de mécanisme de distribution pendant la rotation de la poignée ;
dans lequel l'interaction entre la première partie de mécanisme de distribution et le deuxième mécanisme de distribution affecte une résistance à la rotation (τ) au niveau de la poignée de telle sorte que la résistance à la rotation (τ) alterne entre une résistance à la rotation maximale (τmax) et une résistance à la rotation minimale (τmin) lorsque la poignée est tournée par un utilisateur, et dans lequel la résistance à la rotation indique une position d'alignement de l'ouverture de distribution par rapport à une ouverture de sortie des un ou plusieurs trous du distributeur opérationnel manuellement.

2. Distributeur selon la revendication 1, dans lequel la première partie de mécanisme de distribution a une rigidité variable.

3. Distributeur selon la revendication 1, dans lequel le mécanisme de distribution présente une première résistance à la rotation (τ1) au niveau de la poignée lorsque l'ouverture de distribution est alignée avec un de la pluralité de trous, et une deuxième résistance à la rotation (τ2) au niveau de la poignée lorsque l'ouverture de distribution n'est pas alignée avec un de la pluralité de trous, et (τ2) > (2^{∗}τ1).

4. Distributeur selon la revendication 1, dans lequel 25 N·cm < (τmax) < 150 N·cm.

5. Distributeur selon la revendication 1, dans lequel 0 N·cm < (τmin) < 25 N·cm.

6. Distributeur selon la revendication 1, dans lequel 45 N·cm < (τmax) < 85 N·cm et 2 N·cm < (τmin) < 15 N·cm.
